# EUROPÄISCHE PATENTSCHRIFT

(11) **EP 3 909 492 B1**
(45) Veröffentlichungstag und Bekanntmachung des Hinweises auf die Patenterteilung: **06.09.2023**
(21) Anmeldenummer: 21173722.6
(22) Anmeldetag: 12.05.2021
(51) Int. Cl.: A61L 2/18, A61L 2/22, A61L 2/24, A47L 9/28, A47L 11/40, A47L 13/16, B08B 3/02, B08B 3/08, B05B 7/28, B05B 7/08

(54) **REINIGUNGSMASCHINE MIT DESINFEKTIONSVORRICHTUNG ZUR DESINFEKTION VON OBERFLÄCHEN UND ZUR EIGENDESINFEKTION**
CLEANING MACHINE WITH DISINFECTION DEVICE FOR THE DESINFECTION OF SURFACES AND FOR SELF-DISINFECTION
MACHINE DE NETTOYAGE POURVUE DE DISPOSITIF DE DÉSINFECTION DESTINÉ À LA DÉSINFECTION DES SURFACES ET À SA DÉSINFECTION PROPRE

(30) Priorität: 12.05.2020 DE 102020112873
(43) Veröffentlichungstag der Anmeldung: 17.11.2021
(73) Patentinhaber: ADLATUS Robotics GmbH, 89079 Ulm (DE)
(72) Erfinder: Strobel, Matthias, 89079 Ulm (DE); Hochdorfer, Siegfried, 89079 Ulm (DE)
(74) Vertreter: Meyer, Thorsten

(56) Entgegenhaltungen:
- EP-A1- 1 993 743
- WO-A1-2021/152599

## Beschreibung

Die Erfindung betrifft eine Reinigungsmaschine mit einer Desinfektionsvorrichtung zur Desinfektion von Oberflächen sowohl im zu reinigenden Arbeitsraum als auch in der Reinigungsmaschine selbst.

In diesem Patent wird Desinfektion definiert als chemische Desinfektion die zu einer Reduzierung der auf der zu desinfizierenden Oberfläche vorhandenen Krankheitserreger (Viren und Bakterien) um mindestens 5 log₁₀-Stufen führt, sodass von diesen keine Infektion bzw. Erregerübertragung mehr ausgehen kann.

Aus der EP1993743A1 ist ein Reinigungsgerät bekannt, bei dem eine Selbstdesinfektion vorgeschlagen ist, um die Übertragung von Mikroorganismen von einer Oberfläche auf andere Bereiche eines Gebäudes zu vermeiden. Hierzu sollen Schienen und die Rollen einer desinfizierenden UV-Strahlung ausgesetzt werden.

Aufgabe der Erfindung ist es eine verbesserte Reinigungsmaschine zur Verfügung zu stellen welche zusätzlich zur Reinigung auch eine wirksame Flächendesinfektion und auch eine Eigendesinfektion der Maschine ermöglicht.

Diese Aufgabe wird durch eine Reinigungsmaschine zur Flächenreinigung mit Reinigungseinheit nach den Merkmalen des Anspruchs 1 gelöst.

Erfindungsgemäß ist eine Reinigungsmaschine mit Reinigungseinheit zur Entfernung und Aufnahme von Grob- und/oder Feinschmutz vorgeschlagen, welche so ausgestaltet ist, dass eine Desinfektionseinheit zur Ausbringung von Mitteln zur Desinfektion zur Oberflächendesinfektion und zur Eigendesinfektion von Komponenten der Reinigungsmaschine selbst vorhanden ist. Dem folgend ist die Reinigungsmaschine mit mindestens einem Tank bzw. Behälter und einem Desinfektionsmittelbehälter versehen und die Beimischung des Desinfektionsmittels erfolgt bei Aus- bzw. Einbringung.

Die Reinigungsmaschine kann als reine Reinigungsmaschine sowohl auch als reine Desinfektionsmaschine betrieben werden.

Bevorzugterweise ist vorgesehen die Reinigungsmaschine als Kombinationsmaschine auszuführen, bei welcher zuerst die Reinigung und dann in einem zweiten Schritt eine Desinfektion erfolgt. Diese Desinfektion kann von Vorteil durch Aufbringung des Desinfektionsmittels auf die Bodenfläche erfolgen.

Die Reinigung wird hier vorteilhaft mit einem Reinigungsmittel zur Lösung des Schmutzes in Verbindung mit Wasser ausgeführt. Es ist auch eine reine Reinigung ohne Zusatz von Reinigungsmittel vorgesehen.

Das Desinfektionsmittel selbst wird hierbei in einem geeigneten Behälter oder Tank auf der Reinigungsmaschine bereitgestellt welcher zur Nachfüllung entweder ausgetauscht, nachgefüllt und/oder automatisch in einer Servicestation befüllt werden kann. Typischerweise enthalten Reinigungsmaschinen auch weitere Tanks wie beispielsweise einen Frischwassertank oder einen Tank, in dem das Reinigungsmittel schon beigemischt ist. Hierbei können unterschiedlichste Ausgestaltungen vorkommen wobei als Beispiel auch aus dem Desinfektionsmitteltank Beimischungen zum Frischwassertank erfolgen können oder zur Ausbringung des Desinfektionsmittels zusätzlich noch Wasser aus dem Frischwassertank gemischt wird um die Konzentration des oft in Konzentrat-Form vorliegenden Desinfektionsmittels zu verändern.

Erfindungsgemäß ist vorgesehen, dass die Reinigungsmaschine eine Desinfektionseinheit zur Ausbringung von Desinfektionsmitteln auf die zu reinigende Oberfläche aufweist, wobei hierzu die Desinfektionsmittelkonzentration einer Flüssigkeit aus mindestens einem Behälter auf der Reinigungsmaschine durch hinzudosieren eines höher- und/oder niedriger konzentrierten Desinfektionsmittels aus mindestens einem weiteren Behälter auf der Reinigungsmaschine erfolgt.

Von Vorteil ist nach einer weiteren Ausgestaltung der Erfindung vorgesehen, dass die Desinfektionseinheit zur Ausbringung von Mitteln zur Desinfektion so ausgestaltet ist, dass flüssige, dampf- und/oder gasförmige Desinfektionsmittel durch Einstoff-Drückdüsen wie Kegel- oder Flachstrahldüsen feinzerstäubt oder vernebelt vorzugsweise gleichmäßig auf die zu desinfizierende Fläche aufgetragen wird.

Vorteilhafterweise ist nach einer Ausgestaltung der Erfindung vorgesehen, dass die Desinfektionseinheit zur Ausbringung von Mitteln zur Desinfektion so ausgestaltet ist, dass flüssige, dampf- und/oder gasförmige Desinfektionsmittel durch ein Textil oder eine vergleichbare Komponente (29) mechanisch aufgetragen und/oder flächig verteilt wird.

Von Vorteil ist nach einer Ausgestaltung der Erfindung vorgesehen, dass diese eine Desinfektionseinheit zur Einbringung von Desinfektionsmitteln in den Frischwassertank oder dessen vorgelagerte flüssigkeitsführende Komponenten aufweist, wobei hierzu die Desinfektionsmittelkonzentration der extern eingeleiteten Flüssigkeit durch hinzudosieren eines höher- und/oder niedriger konzentrierten Desinfektionsmittels aus mindestens einem weiteren Behälter auf der Reinigungsmaschine erfolgt.

Von Vorteil ist nach einer Ausgestaltung der Erfindung vorgesehen, dass diese eine Desinfektionseinheit zur Einbringung von Desinfektionsmitteln vor oder in die Reinigungseinheit oder deren vorgelagerten flüssigkeitsführenden Komponenten aufweist, wobei hierzu die Desinfektionsmittelkonzentration einer Flüssigkeit aus mindestens einem Behälter auf der Reinigungsmaschine durch hinzudosieren eines höher- und/oder niedriger konzentrierten Desinfektionsmittels aus mindestens einem weiteren Behälter auf der Reinigungsmaschine erfolgt.

Von Vorteil ist nach einer Ausgestaltung der Erfindung vorgesehen, dass diese eine Desinfektionseinheit zur Einbringung von Desinfektionsmitteln vor oder in die Reinigungseinheit oder deren vorgelagerten flüssigkeitsführenden Komponenten aufweist, wobei hierzu Desinfektionsmittels aus mindestens einem Behälter auf der Reinigungsmaschine erfolgt.

Von Vorteil ist nach einer Ausgestaltung der Erfindung vorgesehen, dass diese eine Desinfektionseinheit zur Einbringung von wahlweise manuell oder automatisch bestimmt Reinigungsmittel oder Desinfektionsmitteln vor oder in die Reinigungseinheit oder deren vorgelagerten flüssigkeitsführenden Komponenten aufweist, wobei hierzu Desinfektionsmittels aus mindestens einem Behälter auf der Reinigungsmaschine erfolgt.

Von Vorteil ist nach einer Ausgestaltung der Erfindung vorgesehen, dass diese über ein Schmutzwassertankreinigungssystem verfügt in welchem während der Tankreinigung und/oder danach Desinfektionsmittel in den Schmutzwassertank oder vorgelagerter flüssigkeitsführender Komponenten eingebracht wird, wobei hierzu Desinfektionsmittels aus mindestens einem Behälter auf der Reinigungsmaschine erfolgt.

Von Vorteil ist nach einer Ausgestaltung der Erfindung vorgesehen, dass diese eine Desinfektionsvorrichtung (28) aufweist, die mit einer oder mehrerer Sprüh- und/oder Vernebelungseinheiten in relevanten Zielbereichen der Reinigungsmaschine, insbesondere die Laufflächen der Antriebs- und passiven Räder und/oder von der Hygiene her wichtige Oberflächen der Reinigungsmaschine selbst ausgestattet ist.

Von Vorteil ist nach einer Ausgestaltung der Erfindung vorgesehen, dass als Desinfektionsmittel Chlordioxid in flüssiger Form in einer hohen Konzentration eingesetzt wird, vorzugsweise in ph-neutraler Form und in Reinform oder hoher Konzentration, vorzugsweise in einer Konzentration, sodass auf der zu desinfizierenden Oberfläche mindestens eine Wirk-Konzentration von 30ppm zur Verfügung steht.

Von Vorteil ist nach einer Ausgestaltung der Erfindung vorgesehen, dass die Reinigungsmaschine als autonomer, d.h. selbstfahrenden Reinigungsroboter realisiert ist.

Weitere vorteilhafte Ausgestaltungen ergeben sich aus den weiteren Unteransprüchen oder deren mögliche Unterkombinationen.

Nachfolgend wird die Erfindung anhand der Zeichnungen weiter erläutert. Im Einzelnen zeigt die schematische Darstellung in:
- Fig. 1: eine schematische Darstellung einer Reinigungsmaschine,
- Fig. 2: eine schematische Darstellung der Flächenzustände,
- Fig. 3a: eine schematische Darstellung einer Reinigungs- und Desinfektionsmittelbeimischung und -ausbringung mit Speisung aus einem gemeinsamen Frischwassertank,
- Fig. 3b: eine schematische Darstellung einer Reinigungs- und Desinfektionsausbringung mit Speisung aus getrennten Reinigungsmittel- und Desinfektionsmitteltanks,
- Fig. 4.: eine schematische Darstellung des Eigendesinfektionsszenarios,
- Fig. 5.: eine schematische Darstellung des Desinfektionsszenarios,
- Fig. 6.: eine schematische Darstellung der Frischwassertankdesinfektion bzw. des Schutzes vor Microfilmbildung, und
- Fig. 7.: eine schematische Darstellung der Schmutzwassertankdesinfektion.

Die in den Figuren gleichen Bezugsziffern bezeichnen gleiche oder gleich wirkende Elemente.

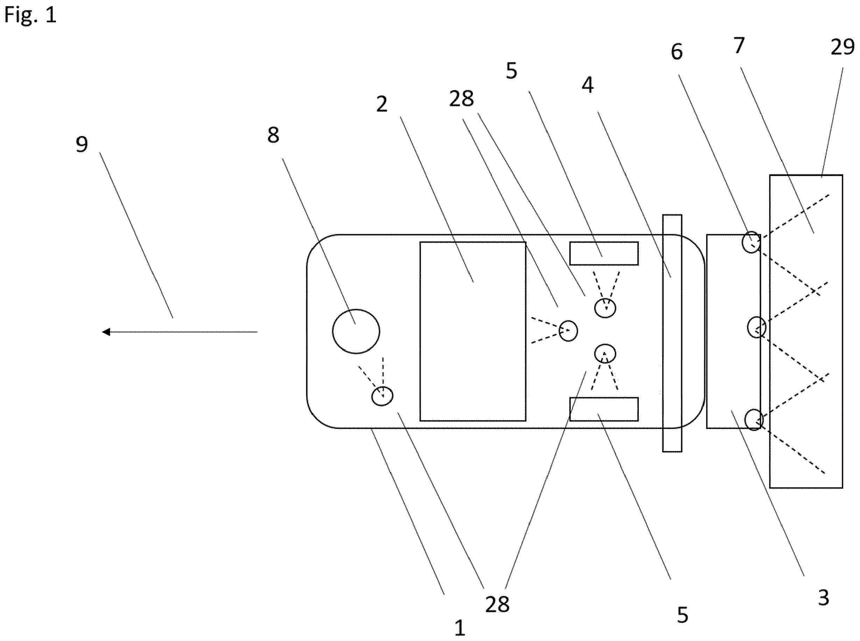

In Fig. 1 ist eine mobile Flächenreinigungsmaschine am Beispiel einer Scheuersaugmaschine in der Draufsicht schematisch dargestellt. Ein mobiles Chassis der Reinigungsmaschine 1 wird hierbei mittels aktiven Antriebselementen bewegt.

Diese Antriebselemente sind hier im Beispiel als sogenannter Differentialantrieb mit 2 aktiven unabhängig ansteuerbaren Antriebsrädern 5 ausgeführt. Ein passives Stützrad 8 dient neben den Antriebselementen 5 der Abstützung des Chassis.

Eine Reinigungseinheit, welche beispielsweise mit einer oder mehrerer rotierenden Bürsten ausgeführt ist, löst den Schmutz der zu reinigenden Fläche mechanisch und optional chemisch mit Zusatz von Reinigungschemie wobei Wasser oder Wasser mit Reinigungsmittel gemischt aus einem Tank der mobilen Flächenreinigungsmaschine ausgebracht wird.

Der Schmutz wird zusammen mit dem ausgebrachten Wasser und der Reinigungschemie durch eine Absaugeinheit Schmutzwasser 4 aufgesaugt so dass in Fahrtrichtung danach eine gereinigte saubere Fläche mit geringer Restfeuchte bleibt.

Die mobile Flächenreinigungsmaschine enthält eine Desinfektionseinheit zur Ausbringung von Mitteln zur Desinfektion 3, welche typischerweise so umgesetzt ist, dass die Ausbringung durch Ausbringelemente für Desinfektionsmittel 6 auf die gereinigte Fläche, d.h. nach der Absaugeinheit, erfolgt. Eine weitere alternative Ausführung der mobilen Flächenreinigungsmaschine ist, die einer sogenannten Kehrmaschine, bei welcher mit rotierenden Bürsten Grob- und Feinschmutz aufgenommen wird. In diesem Fall fällt die Absaugeinheit 4 mit der Reinigungseinheit 2 zusammen. Vorzugsweise ist diese Kehrmaschine mit einer zusätzlichen Saugeinheit versehen, um den Feinschmutz und Staubbildung zu reduzieren.

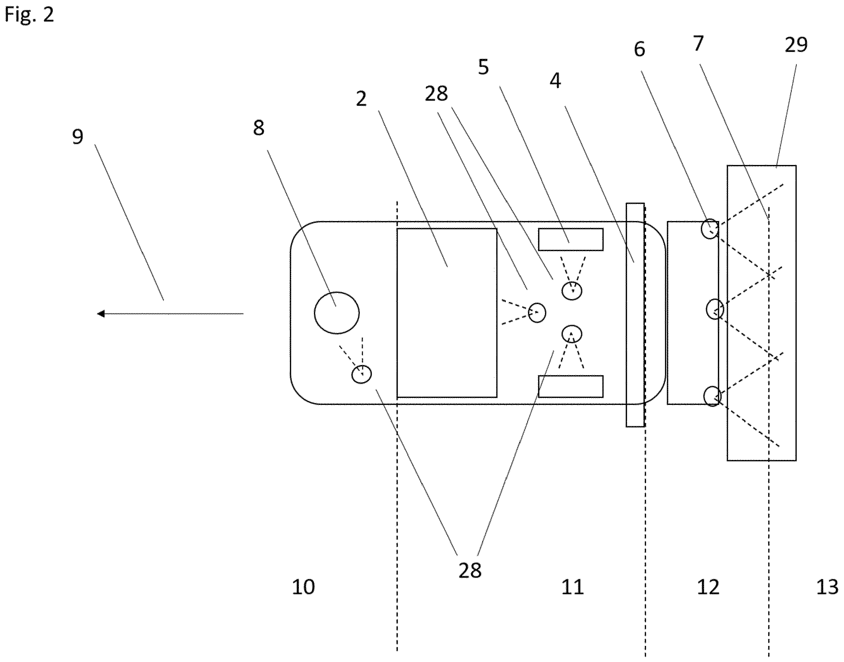

In Fig. 2 sind die Flächenzustände dargestellt. In Fahrtrichtung vor dem Gerät bis hin zur Reinigungseinheit befindet sich die schmutzige Fläche 10. Beginnend von der Reinigungseinheit 2 bis nach der Absaugeinheit Schmutzwasser 4 ist die Fläche mit Wasser und Reinigungschemie benetzt und der Schmutz in Ablösung 11. Nach erfolgter Absaugung von Wasser/Schmutz/Chemie bleibt eine gereinigte Fläche 12. Nach der erfolgten Ausbringung des Desinfektionsmittels bleibt eine mit Desinfektionsmittel benetzte gereinigte und desinfizierte Fläche 13. Die Wirkung des Desinfektionsmittels kann hier einige Zeit in Anspruch nehmen, sodass die Fläche 13 erst nach Ablauf dieser Zeit als desinfiziert betrachtet werden kann.

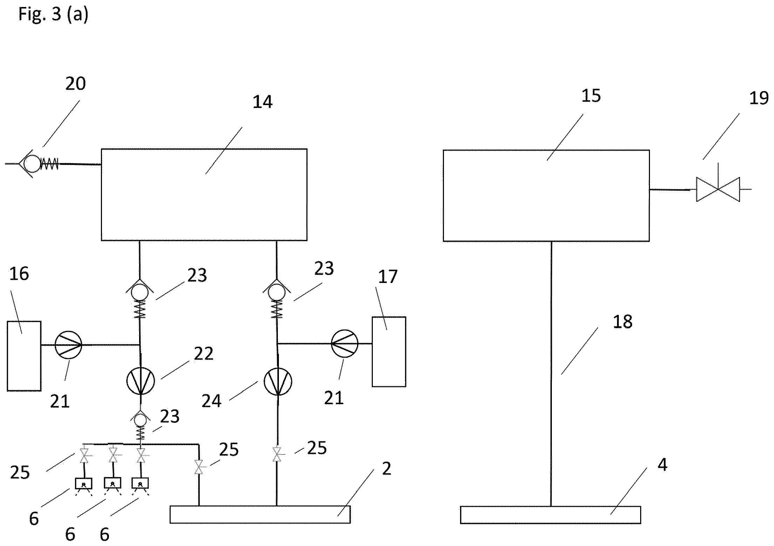

Fig. 3(a) zeigt ein Szenario des Einsatzes einer Reinigungsmaschine, bei welcher eine kombinierte Reinigung und Desinfektion durchgeführt wird.

Hierbei wird für den Teil der Reinigung aus dem Frischwasserbehälter 14 Wasser entnommen und dies mittels einer Dosierpumpe (z.B. Schlauchpumpe) 21 in einem vorgegebenen oder automatisch bestimmten, vorteilhaft variierbaren Mischungsverhältnis mit Reinigungsmittelkonzentrat auf einem Reinigungsmittelbehälter 17 gemischt und dosiert durch eine Pumpe 24 vor der Reinigungseinheit 2 auf den schmutzige Fläche 10 des Fußbodens oder in die Reinigungseinheit 2, typischerweise Bürste, Pad, oder Walze zugeführt. Es ist hierbei auch möglich Frischwasser und Reinigungsmittelkonzentrat separat im jeweiligen Mischungsverhältnis erst am Fußboden oder in der Reinigungseinheit zu mischen. Vorteilhaft ist aber die frühzeitige Mischung zum Beispiel in den Schläuchen wobei die Zumischung typischerweise durch eine Venturi-Düse oder eine Dosierpumpe 21 erfolgt. Weiterhin vorteilhaft ist die Ausführung, in der ein längerer Weg und größeres Volumen nach Beimischung eine bessere Durchmischung von Reinigungskonzentrat und Wasser ermöglicht.

Für den Teil der Desinfektion wird aus dem Frischwasserbehälter 14 Wasser entnommen und dies in einem vorgegebenen oder automatisch bestimmten Mischungsverhältnis durch eine Dosierpumpe 21 (typ. Schlauchpumpe oder Venturidüse) mit dem Desinfektionsmittelkonzentrat aus dem Desinfektionsmittelbehälter 16 gemischt und im Gerät in Fahrtrichtung nach der Reinigungseinheit auf die gereinigte Fläche 12 ausgebracht. Die Ausbringung erfolgt im Beispiel mittels Düsen bzw. geeigneten Ausbringelementen für Desinfektionsmittel 6. Eine alternative Ausbringung kann auch durch Einbringung in ein Flies erfolgen welches die flächige Ausbringung zum zu desinfizierenden Boden ermöglicht.

Mittels einer Absaugeinheit für Schmutzwasser 4 wird typischerweise in mobilen Reinigungsmaschinen über einen Verbindungsschlauch 18 das Schmutzwasser in den Schmutzwassertank 15 abgesaugt und eine gereinigte Fläche 12 mit möglichst geringer Restfeuchte zu hinterlassen. Die Absaugung erfolgt typischerweise über eine Pumpe, welche Vakuum im Schmutzwassertank 15 erzeugt. Das Schmutzwasser des Schmutzwassertank 15 wird manuell oder automatisch über einen Auslassstrang abgelassen.

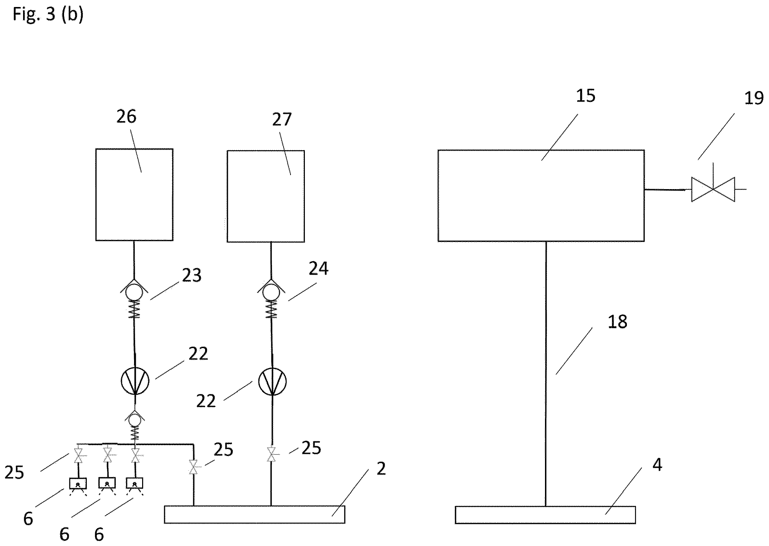

Fig. 3(b) zeigt eine Alternative der Reinigungsmaschine bei welchem sowohl das Reinigungsmittel im auszubringenden Reinigungsmittel-Mischungsverhältnis in einem separaten Reinigungsmitteltank 27 als auch das Desinfektionsmittel im auszubringenden Desinfektionsmittel-Mischungsverhältnis in einem separaten Tank Desinfektionsmitteltank 26 bereitgestellt wird.

Mischformen, wie beispielsweise die Mischung von Wasser aus dem Frischwassertank, Desinfektionsmittel im auszubringenden Desinfektionsmittel- Mischungsverhältnis und Reinigungsmittel in einem separaten Tank, sind ebenso erfindungsgemäß vorgesehen.

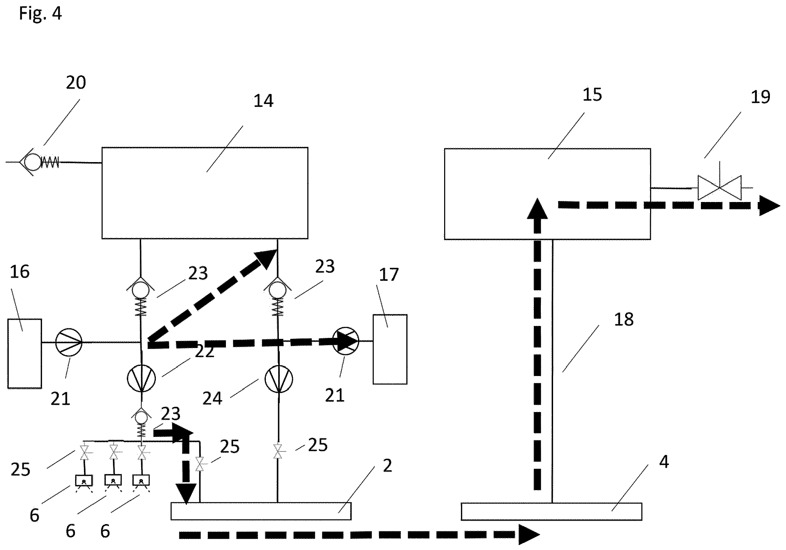

Fig. 4 zeigt ein Szenario des Einsatzes einer Reinigungsmaschine, bei welcher eine Desinfektion durch Ausbringung des Desinfektionsmittel-Mischungsverhältnis in oder vor der Reinigungseinheit freigeschaltet durch ein Ventil 25 durchgeführt wird.

Nach einem weiteren Aspekt der Erfindung wird diese Art der Desinfektion nicht ausschließlich zum Zwecke der Bodendesinfektion eingesetzt sondern dient auch der Desinfektion in einem vorgegebenen oder automatisch bestimmten Desinfektionsintervalls aller Reinigungskomponenten, insbesondere der Reinigungseinheit 2 (Schrubdeck insbesondere Bürsten, Pads und/oder Walzen), Absaugeinheit Schmutzwasser 4 und -schläuche 18, Schmutzwassertank 15 und Schmutzwasserabführungsstrang und -schläuchen/Ventilen 19.

Dies hat den großen Vorteil, dass der komplette Reinigungs- und Schmutzwasserstrang automatisch vollständig desinfiziert werden kann. Dies bietet beim manuellen Einsatz der Reinigungsmaschine die Einsparung der manuellen Desinfektion der Komponenten und ermöglicht beim automatisierten Betrieb, wie dies in einer als Roboter ausgeführten Reinigungsmaschine der Fall ist die automatische Desinfektion.

Im praktischen Einsatz wird hier vorteilhaft für diesen Betriebsfall die Desinfektion auf einer bereits gereinigten und/oder desinfizierten Oberfläche/Boden erfolgen um keine neuen Verunreinigungen während dieses Vorgangs zu bekommen. Eine weitere vorteilhafte Ausführung der Erfindung sieht vor Beimischung des Desinfektionsmittels in der gewünschten Dosierung zur Desinfektion des Gerätes unmittelbar oder nahe dem Wassertank vor um auch das Leitungssystem bis zur Reinigungsmittelbeimischung zu erfassen. Es ist in einer weitern vorteilhaften Ausführung eine zusätzliche Zuführung von Desinfektionsmittel nahe des Reinigungsmittelkonzentrattanks oder -behälters vorgesehen um auch den Teil der Reinigungsmittelführenden Komponenten (typischerweise Schläuche, Dosier-Pumpen und / Ventile) zu desinfizieren.

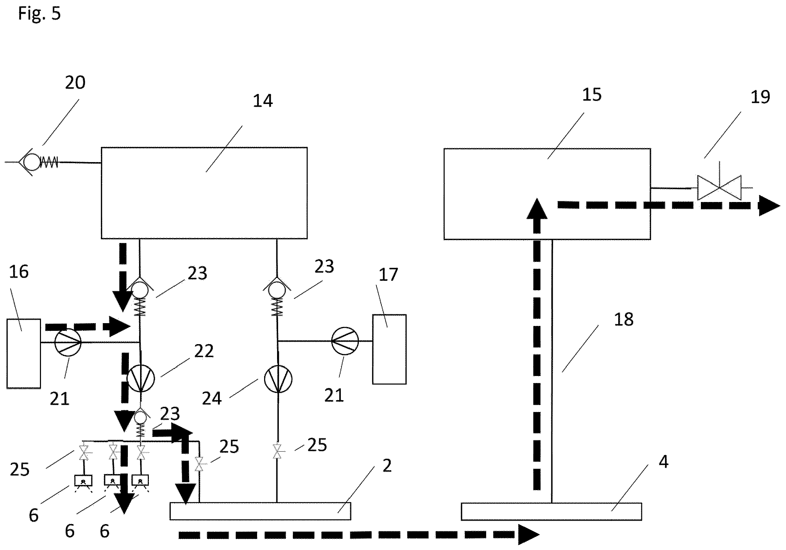

Eine alternative und kombinierte Reinigung/Desinfektion ist in Fig. 5 gezeigt mit einer weiteren vorteilhaften Ausführung einer Reinigungsmaschine, bei welcher eine Desinfektion durch Ausbringung des Desinfektionsmittel-Mischungsverhältnis in oder vor der Reinigungseinheit 2 auf die schmutzige Fläche 10 als auch alternativ oder zeitgleich nach der Reinigungseinheit auf die gereinigte Fläche 12 durchgeführt wird. Dies hat den Vorteil, dass die Reinigungsmaschine zu einem Zeitpunkt als reine Reinigungsmaschine eingesetzt werden kann. Danach oder zu einem späteren Zeitpunkt kann die Maschine als reine Desinfektionsmaschine eingesetzt werden. Ist eine Benetzung oder Ausbringung der Desinfektionslösung nach der Absaugvorrichtung nicht zulässig, kann die Desinfektion hier über die Einbringung in die Reinigungseinheit mit anschließender Absaugung erfolgen, wie dies auch bei der Reinigung erfolgt.

Zur Frischwassertankdesinfektion bzw. zum Schutz vor Microfilmbildung wird nach einem weiteren vorteilhaften Aspekt dieser Erfindung für den Zwecke der vollständigen Desinfektion der Reinigungsmaschine eine für die Desinfektion ausreichende Dosierung des Desinfektionsmittels bei der Speisung des Frischwassertankes mit zugeführt. Dies kann sowohl durch automatische Beimischung bei der Befüllung in einer Servicestation oder am Roboter sowie auch manuell bei der Befüllung oder zu einem späteren Zeitpunkt in den Frischwassertank selbst erfolgen.

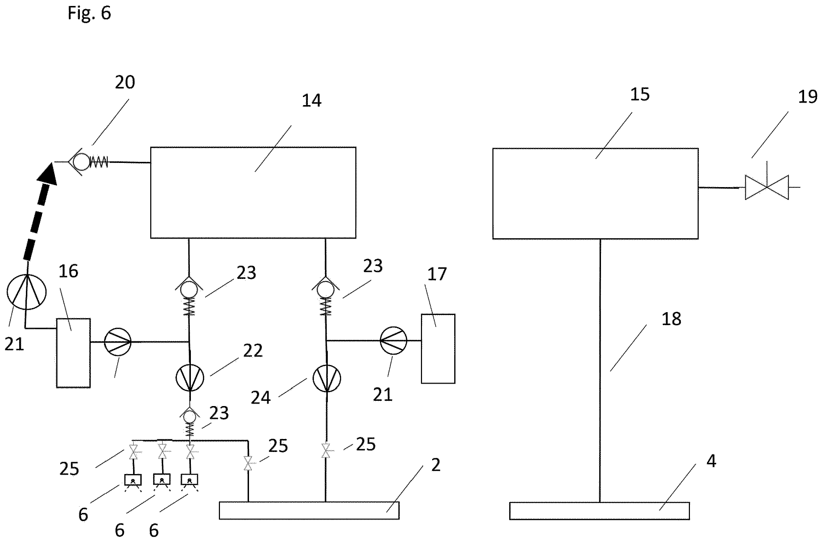

In Fig. 6 ist gezeigt das die Reinigungsmaschine mit einem Schutzsystem für den Frischwassertanksystem 14 auszustatten vorgesehen ist, in welchem Desinfektionsmittel in den Frischwassertank eingebracht wird.

In einer vorteilhaften Ausführung wird Chlordioxid in einer geringen Konzentration während der Frischwasserzufuhr diesem beigemischt. Dies kann sowohl im Reinigungsgerät selbst als auch stationär in der Mischvorrichtung erfolgen oder durch manuelle oder automatische Zumischung im Frischwassertank selbst. Die geringe Konzentration des Chlordioxid ist ausreichend dimensioniert um die Microfilmbildung im Frischwassertank und in den frischwasserführenden Schläuchen, Ventilen, Komponenten zu verhindern und andererseits nicht zu hoch um eine nennenswerte Oxidation des Chlordioxid mit dem Reinigungsmittel und damit in der Regel die resultierende Einschränkung der Wirksamkeit des Reinigungsmittels zu verhindern.

Eine vorteilhafte Ausführung der Beimischungsvorrichtung des Desinfektionsmittels erlaubt eine variable Beimischung, sodass die Konzentration im Bodenreinigungs- und Bodendesinfektionsbetrieb wie oben beschrieben niedrig gehalten werden kann. Im den beschriebenen Einsatzfällen der Eigenreinigung bzw. Desinfektion des Reinigungsgerätes und/oder der Servicestation kann hingegen eine erhöhte Konzentration ausgewählt und realisiert werden.

Für den Einsatzfall in dem eine reine Bodendesinfektion mit dem Reinigungsgerät durchgeführt werden soll kann die hierfür notwendige Desinfektionsmittelbeimischung erfolgen.

In einer vorteilhaften Ausführung wird hierbei eine Chlordioxid in einer Konzentration von 25ppm +/- 10ppm eingesetzt und somit beigemischt.

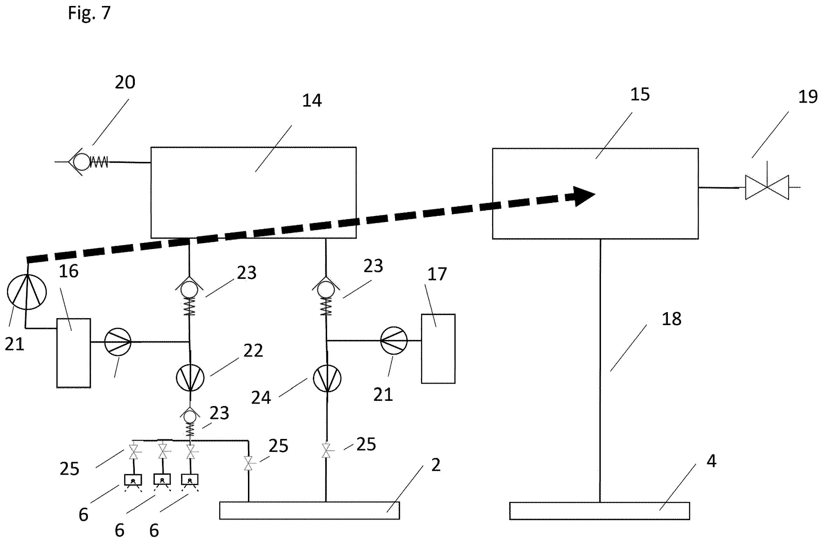

In Fig. 7 ist gezeigt, dass zur Schmutzwassertankdesinfektion die Reinigungsmaschine mit einem Schmutzwassertankreinigungssystem auszustattet ist, in welchem während der Tankreinigung und/oder danach Desinfektionsmittel in den Schmutzwassertank 15 eingebracht wird.

Ein großer Vorteil ist, dass entgegen der aktuell üblichen Vorgehensweise den Schmutzwassertank zu öffnen um die typischerweise unangenehm riechenden und in Teilen schädlichen Gerüche im Raum zu verteilen und demnach am Reinigungsgerät nicht mehr zu intensiv wahrzunehmen.

Nach einem weiteren vorteilhaften Aspekt wird hierbei Chlordioxid der Reinigungsflüssigkeit beigemischt bzw. diese nach erfolgter Reinigung im Tank versprüht und / oder in dessen Volumen vernebelt. Die Konzentration des Chlordioxid wird nach einem weiteren vorteilhaften Aspekt der Erfindung mindestens 20 ppm bei der Mischung mit der Reinigungsflüssigkeit und im Bereich von minimal 1000 ppm bis maximal 5000 ppm, vorzugsweise 1500 ppm bis 3000 ppm, bei reiner Versprühung / Vernebelung im Tank erfolgen.

Vorteilhafterweise ist zur Desinfektionsmischungsbereitstellung für weitere Anwendungsfälle vorgesehen die Reinigungsmaschine mit einem oder mehreren Anbindungen für weiterführende Anwendungsfälle ausgestattet ist, welche die Desinfektionsmittel-Mischung in der für die Anwendung geforderten Konzentration zur Verfügung stellen. Dies können einerseits Anbindungen sein welche zum Beispiel
1. Extern Manuell: Eine manuelle Desinfektionsvorrichtung über einen Schlauch mit einer Ausbringeinrichtung, vorzugsweise eine Sprüh- und/oder Vernebelungseinheit
2. Manipulator: Eine Manipulationsvorrichtung zur Näherung einer Desinfektionsvorrichtung mit einer Sprüh- und/oder Vernebelungseinheit an relevante Zielbereiche in der Umgebung,
3. Eigendesinfektion der Reinigungsmaschine durch Desinfektionsvorrichtung mit einer oder mehrerer Sprüh- und/oder Vernebelungseinheit in relevanten Zielbereichen der Reinigungsmaschine. Dies hat den Vorteil, dass auch Bereiche außer den zuvor beschriebenen Eigendesinfektionsmaßnahmen der Reinigungstechnik selbst erreicht werden können. Dies können zum Beispiel die Laufflächen der Antriebs- 5 und passiven Räder 8 und/oder Oberflächen der Reinigungsmaschine sein.

### Bezugszeichenliste

- 1: Mobiles Chassis der Reinigungsmaschine
- 2: Reinigungseinheit
- 3: Einheit zur Ausbringung von Mitteln zur Desinfektion (im weiteren kurz 'Desinfektionseinheit' genannt)
- 4: Absaugeinheit Schmutzwasser
- 5: Antriebsrad
- 6: Ausbringelemente für Desinfektionsmittel
- 7: Desinfektionsbereich
- 8: Stützrad
- 9: Fahrtrichtung
- 10: Schmutzige Fläche
- 11: Fläche mit Wasser und Reinigungschemie benetzt und der Schmutz in Ablösung
- 12: gereinigte Fläche
- 13: gereinigte und desinfizierte Fläche
- 14: Frischwassertank
- 15: Schmutzwassertank
- 16: Desinfektionsmittelbehälter (Desinfektionskonzentrat)
- 17: Reinigungsmittelbehälter (Reinigungsmittelkonzentrat)
- 18: Verbindungsschlauch Absaugeinheit Schmutzwasser zum Schmutzwassertank
- 19: Auslassstrang Schmutzwassertank
- 20: Einlassstrang Frischwassertank
- 21: Dosierpumpe bzw. Venturiventil für Desinfektionsmittelkonzentrat und Reinigungsmittelkonzentrat
- 22: Pumpe Ausbringmenge Desinfektionsmittelstrang typ. mit Druckerhöhung
- 23: Rückschlagventil
- 24: Pumpe Ausbringmenge Reinigungslösung
- 25: Ventile (typischerweise elektr. Angesteuert)
- 26: Desinfektionsflüssigkeitsbehälter
- 27: Reinigungsflüssigkeitsbehälter
- 28: Einheit zur Ausbringung von Mitteln zur Eigendesinfektion (im weiteren kurz 'Eigendesinfektionseinheit' genannt)
- 29: Vorrichtung zur mechanischen Aufbringung von Desinfektionsmittel

## Patentansprüche

1. Reinigungsmaschine (1), insbesondere eine Scheuersaug- oder Kehrmaschine, zur Flächenreinigung mit Reinigungseinheit (2) zur Entfernung von Grob- und/oder Feinschmutz,
wobei die Reinigungsmaschine eine Desinfektionseinheit (3) zur Ausbringung von Mitteln zur Desinfektion zur Oberflächendesinfektion und zur Eigendesinfektion (28) von Komponenten der Reinigungsmaschine selbst aufweist,
wobei die Reinigungsmaschine eine Desinfektionseinheit (3) zur Ausbringung von Desinfektionsmitteln auf die zu reinigende Oberfläche (7) aufweist, **dadurch gekennzeichnet, dass**
die Reinigungsmaschine derart ausgestaltet ist, dass hierzu die Desinfektionsmittelkonzentration einer Flüssigkeit aus mindestens einem Behälter (16) auf der Reinigungsmaschine durch hinzudosieren eines höher- und/oder niedriger konzentrierten Desinfektionsmittels aus mindestens einem weiteren Behälter auf der Reinigungsmaschine erfolgt.

2. Reinigungsmaschine (1) nach Anspruch 1,
**dadurch gekennzeichnet,**
**dass** die Desinfektionseinheit (3) zur Ausbringung von Mitteln zur Desinfektion (3) so ausgestaltet ist, dass flüssige, dampf- und/oder gasförmige Desinfektionsmittel durch Einstoff-Druckdüsen (6) feinzerstäubt oder vernebelt vorzugsweise gleichmäßig auf die zu desinfizierende Fläche (7) aufgetragen werden.

3. Reinigungsmaschine (1) nach einem der vorhergehenden Ansprüche,
**dadurch gekennzeichnet,**
**dass** die Desinfektionseinheit (3) zur Ausbringung von Mitteln zur Desinfektion (3) so ausgestaltet ist, dass flüssige, dampf- und/oder gasförmige Desinfektionsmittel durch ein Textil oder eine vergleichbare Komponente (29) mechanisch aufgetragen und/oder flächig verteilt werden.

4. Reinigungsmaschine (1) nach einem der vorhergehenden Ansprüche,
**dadurch gekennzeichnet,**
**dass** diese eine Desinfektionseinheit (3) zur Einbringung von Desinfektionsmitteln in den Frischwassertank (14) oder dessen vorgelagerte flüssigkeitsführende Komponenten (20) aufweist, wobei hierzu die Desinfektionsmittelkonzentration der extern eingeleiteten Flüssigkeit durch hinzudosieren eines höher- und/oder niedriger konzentrierten Desinfektionsmittels aus mindestens einem weiteren Behälter (16) auf der Reinigungsmaschine (1) erfolgt.

5. Reinigungsmaschine (1) nach einem der vorhergehenden Ansprüche,
**dadurch gekennzeichnet,**
**dass** diese eine Desinfektionseinheit (3) zur Einbringung von Desinfektionsmitteln vor oder in die Reinigungseinheit (2) oder deren vorgelagerten flüssigkeitsführenden Komponenten (20) aufweist, wobei hierzu die Desinfektionsmittelkonzentration einer Flüssigkeit aus mindestens einem Behälter (16) auf der Reinigungsmaschine durch hinzudosieren eines höher- und/oder niedriger konzentrierten Desinfektionsmittels aus mindestens einem weiteren Behälter auf der Reinigungsmaschine erfolgt.

6. Reinigungsmaschine (1) nach einem der vorhergehenden Ansprüche,
**dadurch gekennzeichnet,**
**dass** diese eine Desinfektionseinheit (3) zur Einbringung von Desinfektionsmitteln vor oder in die Reinigungseinheit (2) oder deren vorgelagerten flüssigkeitsführenden Komponenten (20) aufweist, wobei hierzu Desinfektionsmittels aus mindestens einem Behälter (16) auf der Reinigungsmaschine erfolgt.

7. Reinigungsmaschine (1) nach einem der vorhergehenden Ansprüche,
**dadurch gekennzeichnet,**
**dass** diese eine Desinfektionseinheit (3) zur Einbringung von wahlweise manuell oder automatisch bestimmt Reinigungsmittel oder Desinfektionsmitteln vor oder in die Reinigungseinheit (2) oder deren vorgelagerten flüssigkeitsführenden Komponenten (20) aufweist, wobei hierzu Desinfektionsmittels aus mindestens einem Behälter (16) auf der Reinigungsmaschine erfolgt.

8. Reinigungsmaschine (1) nach einem der vorhergehenden Ansprüche,
**dadurch gekennzeichnet,**
**dass** diese über ein Schmutzwassertankreinigungssystem verfügt, in welchem während der Tankreinigung und/oder danach Desinfektionsmittel in den Schmutzwassertank (15) oder vorgelagerter flüssigkeitsführender Komponenten eingebracht wird, wobei hierzu Desinfektionsmittels aus mindestens einem Behälter (16) auf der Reinigungsmaschine erfolgt.

9. Reinigungsmaschine (1) nach einem der vorhergehenden Ansprüche,
**dadurch gekennzeichnet,**
**dass** diese eine Desinfektionsvorrichtung (3) aufweist, die mit einer oder mehrerer Sprüh- und/oder Vernebelungseinheiten in relevanten Zielbereichen der Reinigungsmaschine, insbesondere die Laufflächen der Antriebs- und passiven Räder und/oder von der Hygiene her wichtige Oberflächen der Reinigungsmaschine selbst ausgestattet ist.

10. Reinigungsmaschine (1) nach einem der vorhergehenden Ansprüche,
**dadurch gekennzeichnet,**
**dass** als Desinfektionsmittel Chlordioxid in flüssiger Form in einer hohen Konzentration einsetzbar ist, vorzugsweise in ph-neutraler Form und in Reinform oder hoher Konzentration, vorzugsweise einer Konzentration größer 1 mg Chlordioxid pro Liter Wasser.

11. Reinigungsmaschine (1) nach einem der vorhergehenden Ansprüche,
**dadurch gekennzeichnet,**
**dass** die Reinigungsmaschine als autonomer, d.h. selbstfahrenden Reinigungsroboter realisiert ist.

## Claims

1. A cleaning machine (1), in particular a scrubbing or sweeping machine, for surface cleaning, comprising a cleaning unit (2) for removing at least one of coarse and fine dirt,
wherein the cleaning machine comprises a disinfecting unit (3) for dispensing disinfectants for disinfecting surfaces and for self-disinfection (28) of components of the cleaning machine itself,
wherein the cleaning machine comprises a disinfecting unit (3) for dispensing disinfectants onto a surface (7) to be cleaned, **characterized in that** the cleaning machine is configured such that the disinfectant concentration of a fluid from at least one container (16) on the cleaning machine is adjusted by metered adding of a disinfectant with a higher and/or lower concentration from at least one other container on the cleaning machine.

2. The cleaning machine (1) of claim 1,
**characterized in that**
the disinfecting unit (3) for dispensing disinfectants (3) is configured such that liquid, vaporized and/or gaseous disinfectants are applied onto the surface (7) to be disinfected by atomizing or nebulizing the disinfectants through single substance pressure nozzles (6).

3. The cleaning machine (1) according to any one of the preceding claims,
**characterized in that**
the disinfecting unit (3) for dispensing disinfectants (3) is configured such that liquid, vaporized and/or gaseous disinfectants are mechanically applied and/or distributed over a surface by means of a fabric or a comparable component (29).

4. The cleaning machine (1) according to any one of the preceding claims,
**characterized in that**
the machine comprises a disinfecting unit (3) for introducing disinfectants into the fresh water tank (14) or into liquid containing components (20) upstream thereof, wherein the disinfectant concentration of the externally supplied liquid is adjusted by metered adding of a disinfectant with a higher and/or lower concentration from at least one other container (16) on the cleaning machine (1).

5. The cleaning machine (1) according to any one of the preceding claims,
**characterized in that**
the machine comprises a disinfecting unit (3) for introducing disinfectants upstream of or into the cleaning unit (2) or into liquid containing components (20) upstream thereof, wherein the disinfectant concentration of a fluid from at least one container (16) on the cleaning machine is adjusted by metered adding of a disinfectant with a higher and/or lower concentration from at least one other container on the cleaning machine.

6. The cleaning machine (1) according to any one of the preceding claims,
**characterized in that**
the machine comprises a disinfecting unit (3) for introducing disinfectants upstream of or into the cleaning unit (2) or into liquid containing components (20) upstream thereof, wherein a disinfectant from at least one container (16) on the cleaning machine.

7. The cleaning machine (1) according to any one of the preceding claims,
**characterized in that**
the machine comprises a disinfecting unit (3) for selectively either manually or automatically introducing cleaning agents or disinfectants upstream of or into the cleaning unit (2) or into liquid containing components (20) upstream thereof, wherein a disinfectant from at least one container (16) on the cleaning machine.

8. The cleaning machine (1) according to any one of the preceding claims,
**characterized in that**
the machine comprises a waste water tank cleaning system, where a disinfectant is introduced into the waste water tank (15) or into liquid containing components upstream thereof during at least one of cleaning of the tank and thereafter, wherein a disinfectant from at least one container (16) on the cleaning machine.

9. The cleaning machine (1) according to any one of the preceding claims,
**characterized in that**
the machine comprises a disinfecting device (3) comprising one or more atomizing or nebulizing units in relevant target areas of the cleaning machine, in particular at least one of the running surfaces of the drive and passive wheels and the sanitarily important surfaces of the cleaning machine itself.

10. The cleaning machine (1) according to any one of the preceding claims,
**characterized in that**
chlorine dioxide may be employed as a disinfectant in liquid form in high concentration, preferably in pH-neutral form, and as a pure substance or in high concentration, preferably in a concentration above 1 mg of chlorine dioxide per one liter of water.

11. The cleaning machine (1) according to any one of the preceding claims,
**characterized in that**
the cleaning machine is implemented as an autonomous, that is, self-propelled cleaning robot.

## Revendications

1. Machine de nettoyage (1), notamment une autolaveuse ou balayeuse, pour le nettoyage de surfaces avec un module de nettoyage (2) pour l'élimination de saleté grossière et/ou fine, dans laquelle la machine de nettoyage présente un module de désinfection (3) pour diffuser des agents de désinfection en vue de la désinfection de surfaces et en vue de la désinfection propre (28) de composants de la machine de nettoyage elle-même,
dans laquelle la machine de nettoyage présente un module de désinfection (3) pour la diffusion de désinfectants sur la surface à nettoyer (7), **caractérisée en ce que** la machine de nettoyage est configurée de telle sorte que la concentration en désinfectant d'un liquide provenant d'au moins un contenant (16) sur la machine de nettoyage s'effectue à cet effet par dosage par addition d'un désinfectant à concentration supérieure et/ou inférieure provenant d'au moins un autre contenant sur la machine de nettoyage.

2. Machine de nettoyage (1) selon la revendication 1,
**caractérisée en ce**
**que** le module de désinfection (3) est configuré pour la diffusion d'agents de désinfection (3) de sorte que des désinfectants liquides, sous forme de vapeur et/ou gazeux sont appliqués de préférence uniformément sur la surface à désinfecter (7) de manière finement pulvérisée ou nébulisée par des buses sous pression pour substance unique (6).

3. Machine de nettoyage (1) selon une des revendications précédentes,
**caractérisée en ce**
**que** le module de désinfection (3) est configuré pour la diffusion d'agents de désinfection (3) de sorte que des désinfectants liquides, sous forme de vapeur et/ou gazeux sont appliqués mécaniquement et/ou répartis de manière plane par un textile ou un composant comparable (29).

4. Machine de nettoyage (1) selon une des revendications précédentes,
**caractérisée en ce**
**que** celle-ci présente un module de désinfection (3) pour l'introduction de désinfectants dans le réservoir d'eau propre (14) ou ses composants de guidage de liquide logés en amont (20), dans laquelle la concentration en désinfectant du liquide introduit de manière externe s'effectue à cet effet par dosage par addition d'un désinfectant à concentration supérieure et/ou inférieure provenant d'au moins un autre contenant (16) sur la machine de nettoyage (1).

5. Machine de nettoyage (1) selon une des revendications précédentes,
**caractérisée en ce**
**que** celle-ci présente un module de désinfection (3) pour l'introduction de désinfectants avant ou dans le module de nettoyage (2) ou ses composants de guidage de liquide logés en amont (20), dans laquelle la concentration en désinfectant d'un liquide provenant d'au moins un contenant (16) sur la machine de nettoyage s'effectue à cet effet par dosage par addition d'un désinfectant à concentration supérieure et/ou inférieure provenant d'au moins un autre contenant sur la machine de nettoyage.

6. Machine de nettoyage (1) selon une des revendications précédentes,
**caractérisée en ce**
**que** celle-ci présente un module de désinfection (3) pour l'introduction de désinfectants avant ou dans le module de nettoyage (2) ou ses composants de guidage de liquide logés en amont (20), dans laquelle de désinfectant provenant d'au moins un contenant (16) s'effectue à cet effet sur la machine de nettoyage.

7. Machine de nettoyage (1) selon une des revendications précédentes,
**caractérisée en ce**
**que** celle-ci présente un module de désinfection (3) pour l'introduction de détergent ou désinfectants déterminés au choix manuellement ou automatiquement avant ou dans le module de nettoyage (2) ou ses composants de guidage de liquide logés en amont (20), dans laquelle de désinfectant provenant d'au moins un contenant (16) s'effectue à cet effet sur la machine de nettoyage.

8. Machine de nettoyage (1) selon une des revendications précédentes,
**caractérisée en ce**
**que** celle-ci dispose d'un système de nettoyage de réservoir d'eau sale, dans lequel du désinfectant est introduit pendant le nettoyage de réservoir et/ou après dans le réservoir d'eau sale (15) ou les composants de guidage de liquide logés en amont, dans laquelle de désinfectant provenant d'au moins un contenant (16) s'effectue à cet effet sur la machine de nettoyage.

9. Machine de nettoyage (1) selon une des revendications précédentes,
**caractérisée en ce**
**que** celle-ci présente un dispositif de désinfection (3) qui est équipé d'un ou plusieurs modules de pulvérisation et/ou nébulisation dans des régions cibles pertinentes de la machine de nettoyage, notamment les surfaces de roulement des roues d'entraînement et passives et/ou surfaces importantes du point de vue hygiénique de la machine de nettoyage elle-même.

10. Machine de nettoyage (1) selon une des revendications précédentes,
**caractérisée en ce**
**que** du dioxyde de chlore peut être utilisé sous forme liquide à une concentration élevée en tant que désinfectant, de préférence sous forme à pH neutre et sous forme pure ou à concentration élevée, de préférence une concentration supérieure à 1 mg de dioxyde de chlore par litre d'eau.

11. Machine de nettoyage (1) selon une des revendications précédentes,
**caractérisée en ce**
**que** la machine de nettoyage est réalisée en tant que robot de nettoyage autonome, c'est-à-dire automoteur.
